# NEW EUROPEAN PATENT SPECIFICATION

(11) **EP 0 479 866 B2**
(45) Date of publication and mention of the opposition decision: **25.05.2005**
(45) Mention of the grant of the patent: 05.04.2000
(21) Application number: 90910520.7
(22) Date of filing: 29.06.1990
(51) Int. Cl.: C12N 15/40, A61K 39/187, C07K 14/185, G01N 33/50, A61K 31/70

(54) **PESTIVIRUS NUCLEOTIDE SEQUENCES AND POLYPEPTIDES, IN PARTICULAR FROM HOG CHOLERA VIRUS**
NUKLEOTIDSEQUENZEN UND POLYPEPTIDE VON PESTIVIRUS, IM EINZELNEN VOM SCHWEINE-CHOLERA-VIRUS
SEQUENCES DE NUCLEOTIDES ET POLYPEPTIDES DU PESTIVIRUS, EN PARTICULIER DU VIRUS DU CHOLERA PORCIN

(30) Priority: 29.06.1989 NL 8901651
(43) Date of publication of application: 15.04.1992
(73) Proprietor: CENTRAAL DIERGENEESKUNDIG INSTITUUT, 8221 RA Lelystad (NL)
(72) Inventor: MOORMANN, Robertus, Jacobus, Maria, NL-8252 EH Dronten (NL); WENSVOORT, Gert, NL-8225 KM Lelystad (NL)
(74) Representative: Winckels, Johannes Hubertus F.
(86) International application number: PCT/NL1990/000092
(87) International publication number: WO 1991/000352

(56) References cited:
- The Journal of Immunology, Volume 141, No. 10, 15 November 1988, The American Association of Immunologists, (US) M.T. SWEETSER et al.: "Class I MHC-Restricted Recognition of Cells Expressing a Gene Encoding A 41 Amino Acid Product of the Influenza Hemagglutinin", pages 3324-3328 see the whole article
- Proc. Natl. Acad. Sci. USA, Volume 86, January 1989, T.J. BRACIALE et al.: "Class I Major Histocompatibility Complex-Restricted Cytolytic T Lymphocytes Recognize a Limited Number of Sites on the Influenza Hemaglutinin", pages 277-281 see pages 280-281
- Viral Immunology, Volume 2, No. 3, 1989, Mary Ann Liebert, Inc. Publishers, K. KUWANO et al.: "Cytotoxic T Lymphocytes Recognize a Cross-Reactive Epitope on the Transmembrane Region of Influenza H1 and H2 Hemagglutinins", pages 163-172 see page 164, lines 3-29; pages 171-172
- Veterinary Microbiology, Volume 23, 1990, Elsevier Science Publishers B.V., (Amsterdam, NL), R.J.M. MOORMANN et al.: "Nucleotide Sequence of Hog Cholera Virus RNA: Properties of the Polyprotein Encoded by the Open Reading Frame Spanning the Viral Genomic RNA", pages 185-192 see the whole article
- Virology, Volume 171, 1989, Academic Press, Inc., T. RUMENAPF et al.: "Hog Cholera Virus Characterization of Specific Antiserum and Identification of cDNA Clones", pages 18-27 see the whole article
- J. Gen. Virol., Volume 70, February 1989, SGM, (GB), M.S. COLLETT et al.: "Review Article. Recent Advances in Pestvirus Research", pages 253-266 see page 256, line 25 - page 257, line 16
- Virology, Volume 171, 1989, Academic Press, Inc., G. MEYERS et al.: "Molecular Cloning and Nucleotide Sequence of the Genome of Hog Cholera Virus", pages 555-567 see the whole article
- Proc. Natl. Acad. Sci. USA, Volume 85, February 1988, E. SZCZESNA-SKORUPA et al.: "Positive Charges at the NH2 Terminus Convert the Membrane-Anchor Signal Peptide of Cytochrome P-450 to a Secretory Signal Peptide", pages 738-742 see page 741, column 2, line 18 - page 742
- WENSVOORT G. epitopes on structural proteins of hog cholera(swine fever)virus,doctoral thesis 03.1989
- WENSVOORT G ET AL. An enzyme immunoassay employing monoclonal antibodies and detecting specifically antibodes to classical swine fever virus, VETERINARY MICROBIOLOGYNL. AMSTERDAM vol17 no pages 129 to 140
- WENSVOORT G: "Epitopes on structural proteins of hog cholera (swine fever) virus", Doctoral thesis, 03.1989
- WENSVOORT G ET AL: "An enzyme immunoassay employing monoclonal antibodies and detecting specifically antibodies to classical swine fever virus", VETERINARY MICROBIOLOGY, NL, Amsterdam, , vol. 17, no. , pages 129 to 140

## Description

The invention relates to nucleotides and polypeptides for providing protection against and diagnosis of pestivirus infections, in particular hog cholera virus infections.

Hog cholera virus (or swine fever virus) in pigs and two other viruses, bovine viral diarrhoea virus (BVDV) in cattle and border disease virus (BDV) in sheep, all belong to one genus, the genus pestivirus in the family of Togaviridae (Westaway et al., Intervirology, 24, 125-139 (1985)); these viruses possess a plus strand, i.e. infectious, RNA genome. Structurally and antigenically, these three viruses are closely related to one other: an antiserum directed against one of the three will generally also react with the other two. BVDV and BDV resemble one another so closely that they are actually regarded as one virus type (Terpstra, in: Progress in Veterinary Microbiology and Immunology. Ed.: R. Pandey, Vol 1, pp. 175-198 (1985)). Although each of the three viruses has its own primary host, BVDV and BDV can also infect other ruminants, and, incidentally, also pigs. A property common to the three viruses is that they can cause congenital infections (Van Oirschot, Veterinary Microbiology 9, 113-120 (1983)). The complete nucleotide sequence of one strain of BVDV is known from EP-A-208672; complete nucleotide sequences of hog cholera virus and BDV are not yet known.

A review of research on pestivirus (including HCV) genomes and proteins is presented in *J. Gen. Virol.* **70**, 253-266 (1989). EP-A-389034, published 26.09.90 and partly based on a priority date of 19.03.89, discloses the nucleotide and amino acid sequence of the HCV Alfort strain.

Vaccination against pestivirus infections is generally carried out using live, attenuated virus strains. Thus, vaccination against hog cholera is usually performed with the aid of specific strains of the hog cholera virus (HCV), for example the so-called Chinese strain. Even though such vaccinations usually provide sufficient protection against hog cholera, multiplication of the vaccine virus strains is often a problem. In addition, the Chinese strain and other vaccine strains can pass through the placenta of pregnant sows after vaccination, and in this way the foetuses can be infected with the vaccine virus. As a result, the foetuses may die or have long-term illness after birth (Overby and Eskildsen, CEC Report Hog Cholera/Classical and African Swine Fever EUR 5904, 420-429 (1976)). The Chinese vaccine virus has been found in the tonsils for up to 15 days after vaccination (Terpstra, Tijdschrift voor Diergeneeskunde, 103, 678-684 (1978)) and can persist longer in swine. This can be confusing if such a vaccinated animal is submitted for diagnosis; differentiation from field virus is possible only after further diagnostic examination.

Antibodies directed against the Chinese strain and also against other HCV vaccine viruses cannot be distinguished from those directed against field virus. Consequently, vaccination of a population of pigs against hog cholera and simultaneous detection of HCV field infections in this population by means of serological screening is impossible.

The known methods for protection of cattle and sheep against BVDV and BDV infections respectively have corresponding drawbacks.

There is therefore a need for improved, simpler means for protection against and diagnosis of pestivirus infections, in particular hog cholera.

The complete RNA nucleotide sequence of the virulent HCV strain Brescia has now been found.

It has furthermore been found that sequences which code for specific parts of structural proteins of an infectious organism are particularly suitable as a base material for protection against the infection. These parts are defined in the claims.

The invention therefore relates to these nucleotide sequences, which are suitable for protection against a pestivirus infection. The invention furthermore relates to recombinant polypeptides encoded by such nucleotide sequences and to a process of preparing such polypeptides by recombinant means. Also, the invention concerns vaccines and diagnostic kits containing such nucleotide sequences or polypeptides. The invention is defined in the appending claims.

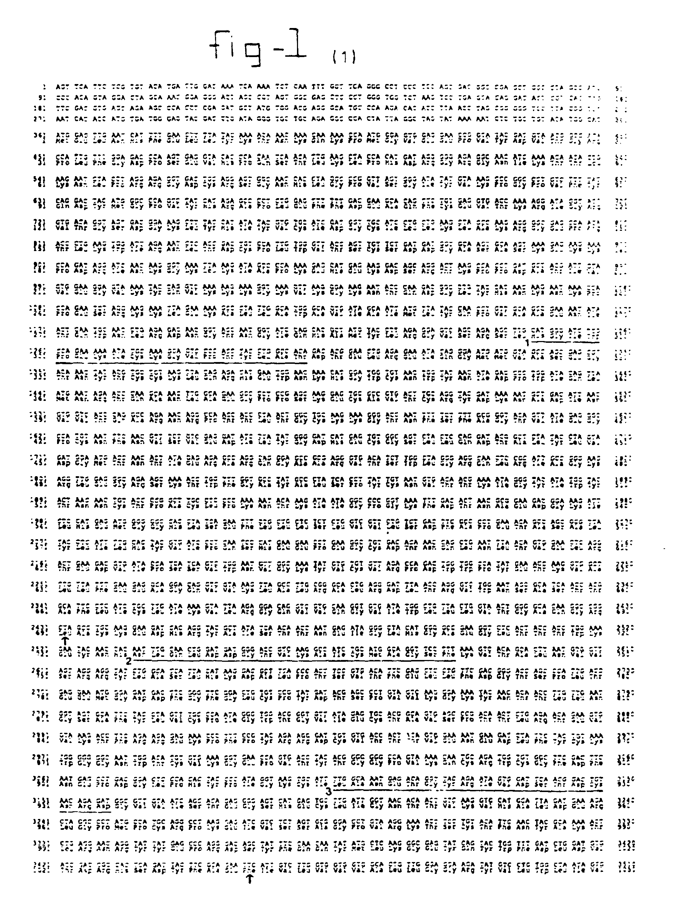

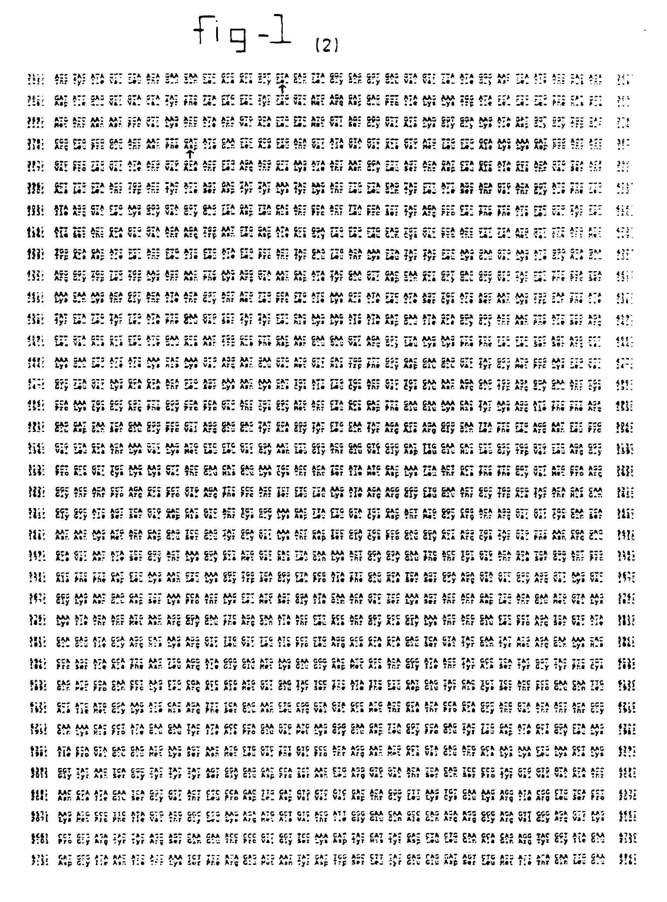

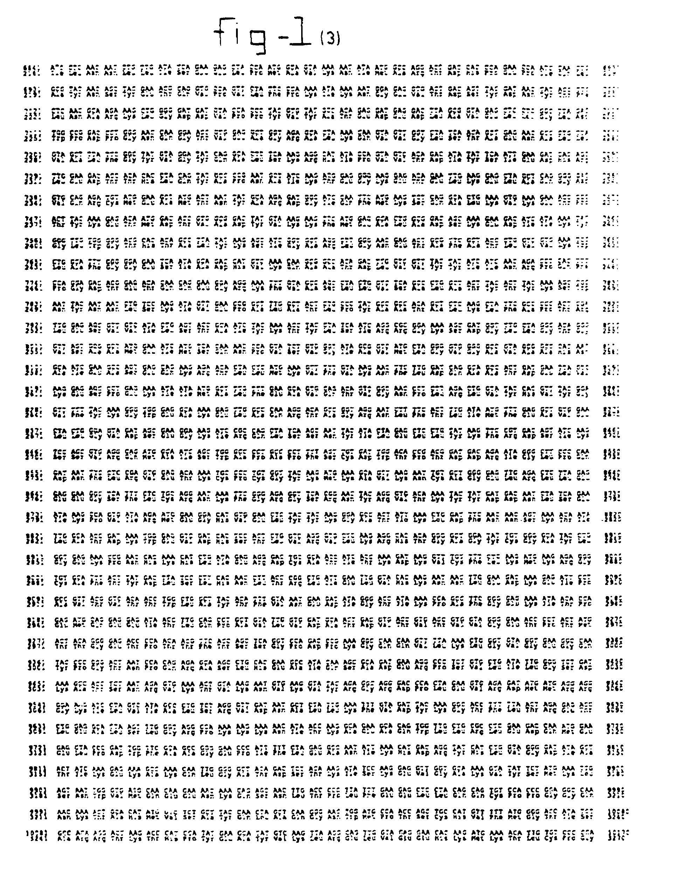

The nucleotide sequence of HCV is shown in Figure 1. The sequence shown is that of the Brescia strain, clone No. 111 (CDI, Lelystad). It has been found that strains of hog cholera virus possess a high degree of serological homology; hog cholera strains cannot be differentiated from one other by means of cross-neutralization tests, but can be differentiated from BVDV (bovine viral diarrhoea virus) and BDV (border disease virus) (Wensvoort et al.., Vet. Microbial., 20: 291-306 (1989); Thesis, University of Utrecht (1989), chapter 3). It has also been found that strains of hog cholera are uniformly recognized by a group of monoclonal antibodies against the envelope protein E1. This group of monoclonal antibodies does not recognize BVDV or BDV strains (Wensvoort et al., Vet. Microbial., 21: 9-20 (1989); Thesis (1989), chapter 4). Conserved epitopes on the HCV envelope protein E1 have been found in all 94 HCV strains investigated (Wensvoort, Thesis 1989, pp. 47 and 78). Sequences of HCV strains other than Brescia which show slight differences with regard to the sequence according to Fig. 1 thus essentially also belong to the invention. In the text which follows, the numerals relate to the genome of HCV strain Brescia; the positions may be slightly different for other strains, but these differences are not significant for the invention. The sequence shown comprises an open reading frame of 11,694 nucleotides which is bordered on either side by a non-coding region. This non-coding region has a length of 360 nucleotides at the 5' end and a length of 229 nucleotides, starting with the stop signal TGA, at the 3' end. The open reading frame is therefore numbered (5')361-12054(3'). The section from nucleotide 361 to 3804 codes for structural proteins. These include a capside protein which is coded for by the region located approximately between 361 and 1162, an envelope protein, also called E2, which is coded for by adjacent nucleotides 1162-2070, a protein (not yet identified in detail), also called E3, which is encoded by adjacent nucleotides 2071-2430, and an envelope protein, also called E1, which is encoded by adjacent nucleotides 2431-3804. The envelope proteins called E2 and E1 thus have the approximate positions 267-570 and 691-1148, respectively, in the amino acid sequence according to Fig. 1.

E1, in particular, appears to be effective in generating antibodies which provide protection against pestivirus infections. Immunodominant subsections of the E1 sequence appear to be present in the region from Leu 691 to Glu 1030, while the hydrophobic region which extends approximately from Phe 1031 to Asp 1148 appears to be another subsection of the E1 sequence which is essential for the protective action. The latter subsection contains three transmembrane regions (TMR), which allow the peptide to be anchored in the cell membrane. It has been found that the presence of at least one such trans-membrane region in the peptide is very important in achieving a high degree of protection against the pestivirus. One TMR is located between residues Phe 1031 and Leu 1063 and is preferably present in the sequence used for protection. Polypeptides which correspond only to one section of E1 and which include a transmembrane region are also suitable for providing protection.

Another sequence that is important for providing protection is a polar charged region which starts at approximately Tyr 1012 and terminates at Phe 1031. This sequence together with the transmembrane region encoded by Phe 1031 up to Leu 1063, as it is in E1, is most preferably present in the sequence used for protection. The sequence encoded by Tyr 1012 - Leu 1063 is a so called stop transfer region (and anchors a protein in the membrane of rough endoplasmatic reticulum). Stop transfer sequences consist of a hydrophobic domain of 20-25 residues (i.e. TMR) and a polar region of 10-15 residues (i.e. Tyr 1012-Phe 1031) (E. Perara and V.R. Lingappa, R.C. Das and P.W. Robbins, Eds. (Academic Press, New York, 1988), pp. 3-47). This sequence may also be coupled to another antigen encoded by the pestivirus RNA (i.e. E2, E3, p80), or to a heterologous antigen (i.e. bacterial toxin, peplomer antigen of a coronavirus, i.e. TGE, Haemagglutinine of an influenza virus, i.e. porcine influenza virus, etc. etc.) and thereby increase the immunogenicity of such an antigen (irrespective of the expression system). Also the TMR apart from the polar region may be used, or the polar region apart from the TMR. TMR and polar region may also be used in conjunction with a heterologous polar region and TMR respectively. The use of a stop transfer region is not limited to such regions derived from a pestivirus. Further, the immunogenicity of a pestivirus antigen, but also of other antigens, can also be increased when the sequence encoding the antigen is coupled to a heterologous stop transfer region or part thereof.

The term polypeptides used here is to be understood to mean oligopeptides and polypeptides. The polypeptides can optionally be glycosylated or modified in some other way.

Important nucleotide sequences for protection according to the invention are therefore those which code for the envelope protein E1, and sections thereof, including stop transfer sequence. The sequence according to the invention therefore preferably contains at least one section as defined in the claims of the nucleotide sequence (5') 1-3804 (numbering according to Fig.1). The said areas are valid for HCV; for BVDV and BDV their positions are somewhat different, but the general arrangement is the same; the sequences of the corresponding envelope proteins and sections thereof are also particularly useful for protection and diagnostics.

The importance of the structural proteins and of polypeptides completely or partially corresponding thereto for the protective action against pestivirus infections is evident from the fact that pigs inoculated with a recombinant virus comprising a cDNA sequence which codes for a structural protein of HCV, in particular E1, produce antibodies against E1, form neutralizing antibodies against HCV and are protected against infection with virulent hog cholera virus.

The invention furthermore relates to recombinant RNA and DNA in which one or the sequences described above is incorporated; a recombinant polynucleotide of this type has, for example, the form of a recombinant virus. A suitable carrier virus for this is the pseudo-rabies virus (PRV), but other viruses, such as vaccinia virus, adenovirus, baculovirus, SV40, retrovirus, hepatitis B virus and derivatives thereof, can also be used. Cells which have undergone stable transformation with one of these viruses, cells in which these viruses replicate episomally or lytically and cells which have undergone stable transformation with suitable vectors and express the structural protein, in particular the E1 protein or a part thereof sub-units as defined in the claims optionally obtained via recombinant DNA in prokaryotic or eukaryotic systems, may furthermore be used. Such a recombinant system is exceptionally suitable as a basis for a vaccine against a pestivirus infection, in particular against HCV. The invention therefore also relates to vaccines which contain the nucleotide sequence in a recombinant virus or in another form.

The invention further includes vaccines which contain the polypeptides, as described above.

The invention furthermore relates to means for diagnosis of an infection, in which an antigenic polypeptide as described above, is used, as well as to kits containing such means as essential component.

A particularly useful diagnosis is that in which it is possible to differentiate serologically between an animal vaccinated according to the invention and an infected animal, in particular with HCV. In this case, a polypeptide other than that which generates the protective antibodies is used: the serum of infected animals then contains antibodies against this second polypeptide, while the serum of vaccinated animals does not. The E2 protein is a suitable polypeptide for this purpose.

The following describes methods for culturing the viruses, isolating and cloning RNA, determination of the sequence and preparation of peptides and antisera.

### Cells and viruses

SK-6 cells [Kasza et al., Research Vet. Sc. 13 46-51 (1979)] were grown in Eagle basal medium containing 5% foetal bovine serum (FBS). FBS was irradiated with gamma radiation and subsequently examined thoroughly for BVDV by prolonged growth of foetal bovine epithelial cells (FBE) in media containing such sera. The cells were tested for BVDV in a sensitive immunoperoxidase monolayer assay (IPMA) (Wensvoort et al.. Vet. Microbiol. 12, 101-108 (1986)). FBS was also checked for BVDV antibodies by measurement of the neutralization index of a standard BVDV stock in an IPMA. The only sera used were those which were free from BVDV and contained no BVDV antibodies or had a low BVDV antibody titre.

The HCV Brescia strain was not cytopathogenic and was biologically cloned by means of a threefold end point dilution. After three amplification steps a cloned virus stock (Brescia, clone No. 111) having a titre of 3.10⁷ TCID₅₀/ml was obtained.

### Isolation of intracellular RNA from HCV

Monolayers of SK-6 cells in 175 cm² bottles (Nunclon) were infected with HCV at a multiplicity of infection (MOI) of 10 TCID₅₀ per cell. They were incubated at 37°C for 1.5 hours, and thereafter fresh medium was added to a final volume of 40 ml. After 7 hours, actinomycin D was added to give a final concentration of 1 µg/ml. 24 hours after infection the cells were washed twice with ice-cold PBS. The cells were lysed in an ice-cold lysis buffer (50 mM Tris-HCl, pH 8.2, 0.14 M NaCl, 2 mM MgCl₂, 5 mM DTT, 0.5% (v/v) NP-40, 0.5% Na deoxycholate and 10 mM vanadyl ribonucleoside complexes (New England Biolabs)).

The lysates were centrifuged (4°C, 5 mm, 4000 rpm) and the supernatant was treated with proteinase K (250 µg/ml, final concentration) at 37°C for 30 minutes, extracted twice with phenol, chloroform and isoamyl alcohol (49:49:2) and once with chloroform and isoamyl alcohol (24:1). Intracellular RNA was obtained by precipitation with ethanol, purified via a 5.7 M CsCl cushion and enriched with HCV-specific RNA in a denaturing, isokinetic sucrose gradient of 5-29% (w/v) containing 50% formamide. The sucrose gradient was centrifuged at 200,000 x g for 16 hours in a Beckmann SW40 Ti rotor at 5°C (Moormann and Hulst, Virus Res. 11, 281-291 (1988)).

### Isolation of viral RNA from HCV

Brescia virus was purified essentially as described by Moormann and Hulst (see above). Confluent monolayers of SK-6 cells were infected with a MOI of 0.5, and were grown in 200 ml of medium at 37°C for 48 hours. Cells were subsequently suspended in 50 ml of medium and freeze-dried twice. The cell suspension was clarified and the supernatant was brought onto 0.5 H NaCl and 10% m/v polyethylene glycol 6000 (BDH). The virus was pelleted by centrifuging for 20 minutes at 6000 x g at 4°C, dissolved in TNE (20 mM Tris-HCl, pH 7.2, 150 mM NaCl, 1 mM EDTA) and purified in a gradient of 30-50% glycerol/(0-50%) Na-K tartrate. The gradient fractions were titrated to determine whether virus was present in an IPMA. The fractions suitable for use were combined, and the virus was pelleted by centrifuging for 4 hours at 150,000 x g at 4°C. Viral RNA was extracted from the pellets with ice-cold lysis buffer and proteinase K, as described above. The viral RNA was recovered by precipitation with ethanol.

### Cloning of HCV RNA

Enriched intracellular HCV RNA (approximately 1.5 µg HCV-specific RNA) was incubated with 10 mM methylmercury hydroxide for 10 minutes at room temperature. The denatured RNA sample was incubated at 42°C with 50 mM Tris-HCl, pH 7.8, 10 mM MgCl₂, 70 mM KCI, 0.5 mM dATP, dCTP, dGTP and dTTP, 0.6 µg calf thymus oligonucleotide primers pd(N)6 (Pharmacia) and 300 units of Moloney murine leukaemia virus reverse transcriptase (Bethesda Research Laboratories) in a total volume of 100 µl. 20 mM EDTA was added after one hour; the reaction mixture was then extracted with phenol/chloroform, passed through a Sephadex G50 column and precipitated with ethanol.

For synthesis of the second cDNA strand, DNA polymerase I (Boehringer) and RNAse H (Pharmacia) were used (Gübler arid Hoffman, Gene 25, 263-269 (1983)).

The double-stranded cDNA was incubated with T4 DNA polymerase (Pharmacia) in a reaction mixture which contained 0.05 mM deoxynucleotide-triphosphates and methylated with EcoRI methylase (New England Biolabs), EcoRl linkers (New England Biolabs) were added to the blunt ends of the cDNA with T4 DNA ligase (Pharmacia). After digestion with EcoRI (New England Biolabs), cDNA was separated on a 0.8% agarose gel. Fragments of 1 kb to 4 kb were electroeluted, ligated in the EcoRI site of pUC 19 and used for transformation of the E. coli strain JM 83 (Vieira and Messing, Gene 19, 259-269 (1982)), as described by Hanahan, J.Mol.Biol. 166, 577-580 (1983). Colony filters were hybridized with a ³²P-labelled single-stranded cDNA probe. The probe was reverse transcribed from HCV RNA which had been fractionated on a neutral agarose gel (Moormann and Hulst, see above). Before use the single-stranded DNA probe was incubated with the cytoplasmic RNA from mock-infected SK-6 cells.

The relationship between HCV-cDNA clones was determined by restriction enzyme analysis and by hybridization of Southern blots (Southern, J.Mol. Biol. 89, 503-517 (1975)) of the digested DNA with nick-translated cDNA probes (Maniatis et al., "Molecular Cloning, A Laboratory Manual", Cold Spring Harbor Lab., Cold Spring Harbor, N.Y. 1982).

Clones isolated from the cDNA library described above represent 90% of the HCV RNA. However, RNA sequences at the 5' end and 3' end were not cloned.

In order to obtain cDNA clones of the 3' end, 20 µg viral RNA was polyadenylated in vitro with E. coli polyApolymerase (Pharmacia) in 300 µl of a reaction mixture containing 50 mM Tris-HCl, pH 7.8, 2.5 mN MnCl₂, 10 mM MgCl₂, 250 mM NaCl, 5 mM DTT, 800 µg/ml BSA, 180 units of RNasin (Promega Biotec), 0.2 mM ATP, 4 µCi ³H-ATP and 2 units of polymerase. The reaction lasted 90 minutes at 37°C and was terminated by extraction with phenol (see above). RNA adenylated with polyA was recovered with ethanol and synthesis of the first strand cDNA was primed with oligo-dT (12-18) (Pharmacia). In order to obtain cDNA clones of the 5' end, a synthetic oligonucleotide which is complementary to the nucleotide sequence 308-347 (Fig. 1) was synthesized and used to prime first strand cDNA synthesis on template viral RNA.

The conditions for the synthesis of the first and second strand of cDNA from the 5' and 3' ends were as described above.

Double-stranded cDNA was incubated with T4 polymerase (see above), fractionated on a 0.8% agarose gel and ligated into the Smal site of pGEM-4 blue (Promega Biotec). Ligated DNA was used for transformation of E. coli oH5-α (Hanahan, in DNA Cloning Vol. I; A Practical Approach. Chapter 6, pp. 109-135, 1985).

### Sequencing of cDNA clones

Sequence analysis was largely carried out after subcloning of suitable restriction fragments in M13mp18 and mp19 (Yanisch-Perron et al., Gene 33, 103-119, (1985)). The remaining section of the sequence was determined by direct sequence determination of double-stranded DNA of plasmid clones with a sequence kit based on T7 polymerase (Pharmacia). In addition to the universal M13 primer of 15 nucleotide length (Boehringer), sequencing was also carried out using oligonucleotide primers prepared on a cyclone DNA synthesizer (New Brunswick Scientific). The nucleotide sequences of the subcloned HCV-specific restriction fragments were determined by means of the dideoxy chain termination method (Sanger et al., Proc. Natl. Acad. Sci. USA 74, 54-63, 54-67 (1977)) using Klenow DNA polymerase (Pharmacia) or reverse transcriptase (Life Sciences) (Sanger et al., see above; Smith, DNA Sequence Analysis by Primed Synthesis in "Methods in Enzymology", Vol. 65, pp. 560-580, Academic Press, New York) and α-³⁵S-dATP (Amersham).

The sequencing reactions were analysed on a 6% acrylamide gel with 8M urea. The data were analysed with a M24 computer from Olivetti using a PCGene sequence analysis program (Genofit, Geneva, Switzerland) and with a Macintosh SE computer from Apple using DNA "Strider" (Merck, Nucl. Acids Res. 16, 1829-1836 (1988)).

### Preparation of synthetic peptides and preparation of antipeptide sera in rabbits.

Peptides were synthesized by known methods using a solid phase and BOP [benzotriazol-1-yloxytris(dimethylamino)phosphonium hexsfluorophosphate] reagent (Fournier et al., Int.J.Peptide Protein Res. 31, 86-97 (1988)).

Antisera against peptides were prepared by immunizing rabbits with 1 mg peptide conjugated with keyhole lympet haemocyanin (KLH) (KLH:peptide = 1:1) in complete Freund's adjuvant. The rabbits were bled 70 days after immunization.

### Analysis of antipeptide sera using Western blots

The envelope protein E1 of HCV Brescia was purified by means of immunoaffinity, fractionated by sodium dodecylsulphate/polyacrylamide gel electrophoresis and transferred to nitrocellulose paper (blots) as described by Wensvoort (thesis: Epitopes on Structural Proteins of Swine Fever (Hog cholera) Virus. University of Utrecht, pp. 71-85). The blots were soaked for 30 minutes at 37°C in PBS + 0.85 M NaCl + 0.05% Tween 80 and 5% horse serum (blotting buffer), and were then incubated for 60 minutes at 37°C with the antipeptide sera, diluted 1:10 in blotting buffer. After washing four times in PBS + 0.05% Tween 80, bound rabbit immunoglobulins were detected by a second incubation for 60 minutes at 37°C with a HRPO conjugate directed against rabbit IgG and IgM. The blots were then washed as above and incubated with substrate solution as described by Wensvoort (see above). As positive controls, E1 blots were stained with concanavalin-A and with monoclonal antibody-HBPO conjugate 8 (Wensvoort, see above).

### Expression of E1 in cell culture.

Monolayer's of 3 x 10⁶ SK-6 cells were infected with HCV, M203, M204, M205, or M206 at a multiplicity of infection of 10. Cells were labeled with ³⁵S from 6 to 18 hours after infection by incubation in 2 ml cysteine-free Eagle Basal medium, supplemented with glutamine, antibiotics, 5% dialysed foetal calf serum and 100 µCi of [³⁵S]cysteine per ml. At 18 hours after infection cells and supernatants were harvested separately. Cells were lysed in 1 ml PBS-TDS (1% (v/v) Triton X-100, 0.5% (w/v) Na-deoxycholate, 0.1% (w/v) Na-dodecyl-sulphate in phosphate buffered saline). Lysates were sonicated and clarified by centrifugation for 10 min at 80,000 g. Supernatants were clarified by centrifugation for 2 hours at 80,000 g. One hundred microliter samples of lysates or 200 µl samples of supernatants were incubated for 16 hours at 4°C with 2 µl of a 1:1:1 mixture of E1-specific MAbs 3, 6, and 8 (Wensvoort, J. Gen. Virol. 70, 2865 (1989)). These mixtures were then incubated for 2 hours at 4°C with 1 mg protein A sepharose and centrifuged for 1 min at 1000 g. The precipitated proteins were washed four times with PBS-TDS, incubated for 5 min at 100°C in sample buffer (60 mM tris-HCl pH 6.8, 2% Na-dodecyl sulphate, 10% glycerol, 5% 2-mercaptoethanol, 0.01% bromophenol blue), and analysed by electrophoresis on 10% SDS-polyacrylamide gels.

### Description of the figures

### Fig. 1

Complete nucleotide sequence of the DNA copy of the hog cholera virus RNA of strain Brescia.

The methionine residue encoded by nucleotides 361-363 is the first amino acid of the open reading frame which in the sequence extends from nucleotide 361-12054.

The underlined areas indicate the sequence of synthetic peptides (18-mers) 1-3 which are used for preparation of peptide antisera in rabbits (see above).

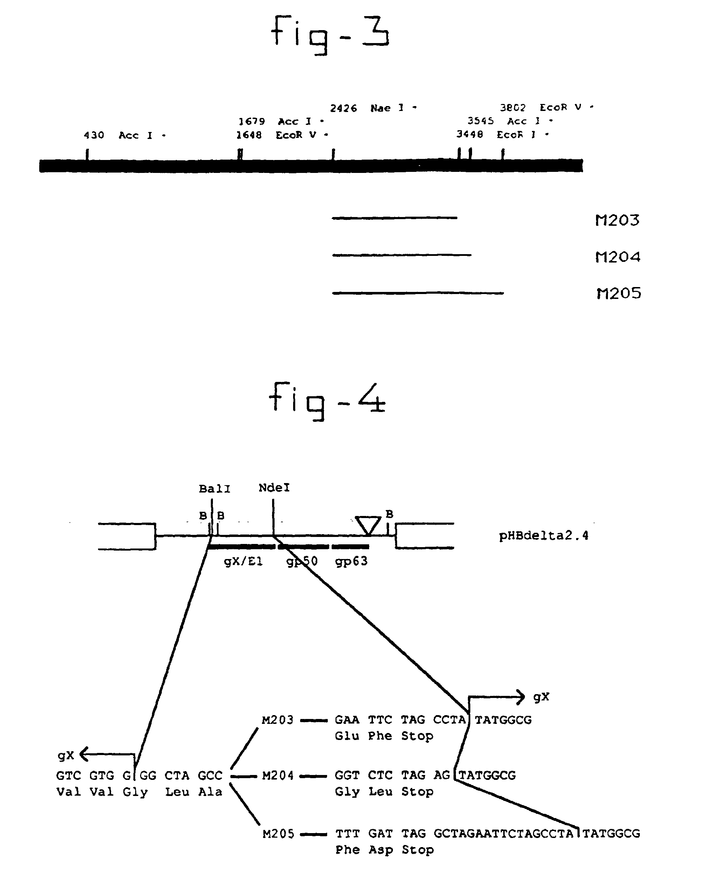

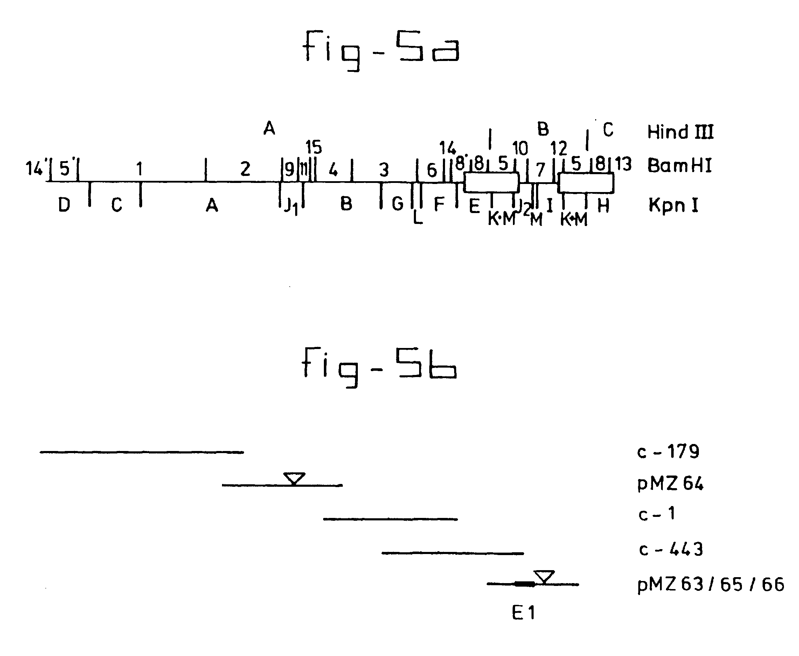

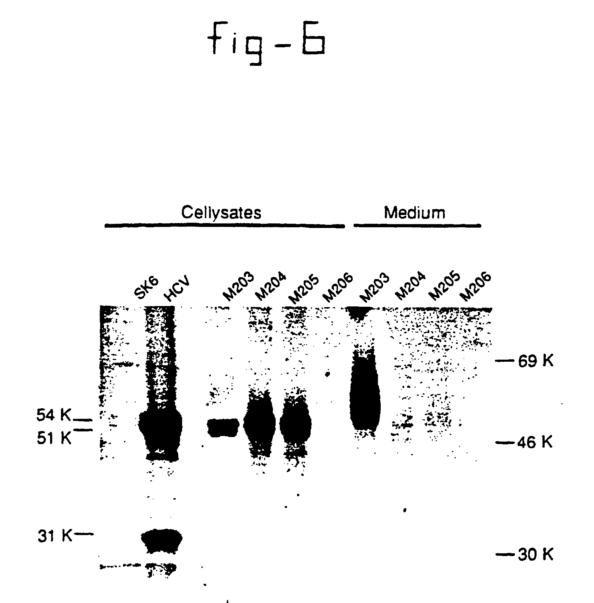

The vertical arrows indicate Leu at amino acid position 691, Phe at position 1031, Leu at position 1063 and Asp at position 1148 (see also Fig. 4).

### Fig. 2

Western blot analysis of rabbit antisera, prepared against peptides 1-3 (see Fig. 1).

The blots were analysed with (from left to right): concanavalin-A, monoclonal antibody 8, (Wensvoort, 1989, see above); antisera against peptides 1-3.

The doublet gp54-51 forms the E1 glycoprotein fraction of HCV strain Brescia; the nature of gp31 has not yet been established.

### Fig. 3

Map of nucleotide 1-4440 of the sequence shown in Fig. 1 for the restriction enzymes Accl, EcoRI, EcoRV and Nael. The position in the sequence (Fig. 1) of the first nucleotide of a recognition site for a restriction enzyme is indicated.

The sites of the E1 restriction fragments M203 (NaeI-EcoRI). M204 (Nael-Accl), and M205 (NaeI-EcoRV) in this sequence are underlined. These fragments were subcloned in pHBdelta2,4 (see Fig. 4).

### Fig. 4

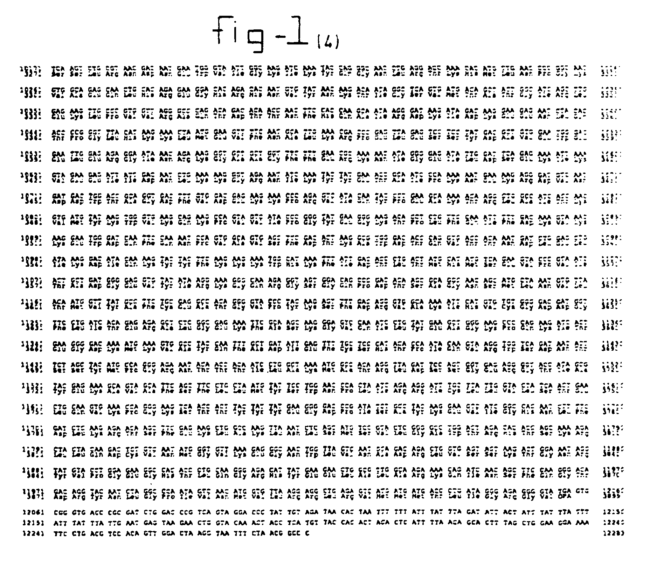

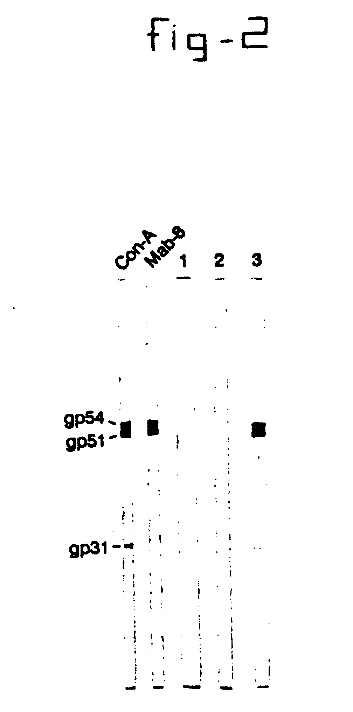

Construction of recombinants between HCV-E1 and pHBdelta2,4. The construction of pHBdelta2,4 has been described by Quint et al., J.Gen.Virol. 68, 523-534 (1987). The triangle indicates a deletion of approximately 2.05 kb in the unique short region of pHBdelta2,4 (Van Zijl, to be published). BamHI sites are indicated with a B. The sequence of gX has been described by Rem et al., J. Virol. 54, 21-29 (1985), the sequence of gp50 by Petrovskis et al., J. Virol. 59, 216-223 (1986) and the sequence of gp63 by Petrovskis et al., J. Virol. 60, 185-193 (1986). Recombinants were constructed by deletion of the restriction fragment Ball-Ndel from pHBdelta2,4. Said restriction fragment comprises most of the coding region (1380 nucleotides) of gX (Rea et al., see above). The Ndel site was filled in with Klenow polymerase, and E1 sequences M203, M204 and M205, shown in Fig. 3, were inserted in pHBdelta2,4.

At the 5' end all E1 sequences possess a blunt end (of Nael) starting with GG. This end is directly ligated at the blunt end, generated after BalI digestion, of pHB-delta2,4. The fusion at the 5' end leads to a Gly codon at the ligation site in all pHBdelta-2,4-E1 constructs. The Leu codon downstream of this Gly codes for amino acid number 691 in the HCV sequence (Fig. 1).

At the 3' end, stop codons were inserted in the constructs by use of specific oligonucleotide linkers. The codon lying directly upstream of the stop codon in construct M203 codes for amino acid 1031 in the HCV sequence (Fig. 1). In constructs M204 and M205, these codons code for amino acids 1063 and 1148, respectively (Fig. 1).

The starting points of the gX sequences alongside the E1 inserts at the 5' and 3 ends are indicated by arrows. The stop codons and sequences up to the gX starting points at the 3' ends of the constructs are derived from the oligonucleotide linkers used.

The molecular cloning procedures were carried out as described by Maniatis et al. (1982, see above).

### Fig. 5

Regeneration of pseudorabies (PRV) recombinant virus from overlapping subgenome PRV fragments (Van Zijl et al., J.Virol. 62, 2191-2195 (1988)).

pMZ66 contains E1 fragment M203, pMZ65 contains E1 fragment M204 and pMZ63 contains E1 fragment M205.

The positions of the transfected clones on the physical map of PRV strain NIA-3 (see 5a) are indicated by vertical bars (see 5b).
(a): physical map of NIA-3 (Quint et al., J.Gen.Virol. 68, 523-534, (1987)). The genome consists of a linear double-stranded DNA molecule which can be divided into a unique short region within inverted repeats (indicated by open rectangles) and a unique long region. The sites of BamHI, HindIII and KpnI are shown.
(b): series of five overlapping clones.

The cosmid clones c-179, c-1 and c-443 have been described by Van Zijl et al., (see above). Clone pMZ64 contains the BgIIl B fragment of strain 783 (Moormann et al., Abstract 12th Int. Herpes Virus Workshop, p. 255 (1987)) in a plasmid vector. The Tk gene in strain 783 (pMZ64) has been inactivated by introduction of a deletion of 19 base pairs (Moormann et al., see above). The position of this deletion in pMZ64 is indicated by a triangle. The construction of clones pMZ63/65 and 66 is described in Fig. 4. Regeneration of PRV-E1 recombinants was carried out as described by Van Zijl et al. (see above). Corresponding to the inserted E1 area (Figures 3 and 4), the recombinant PRV-E1 strains were designated by PRV-M203, PRV-M204 and PRV-M205.

The construction of PRV-M206 was identical to that of strains PRV-M203 to M205 inclusive; strain PRV-M206 is identical to said strains but lacks the HCV-specific E1 sequence.

### Fig. 6

Expression of the E1 protein in cell culture.

Autoradiograph of an SDS-polyacrylamide gel, showing immunoprecipitates from lysates and supernatants (medium) of M203-, M204-, M205-, and M206-infected cells with a mixture of E1-specific MAbs. Wild-type E1 (molecular mass of 51 to 54 kD), precipitated from lysates of HCV-infected cells, was run in parallel. A 31-kD glycoprotein is always coprecipitated with E1.

### Example

The tests below were carried out to determine whether, as a result of vaccinating pigs with one of the three PRV constructs M203, M204 and M205 or with the PRV construct M206, the pigs formed neutralizing antibodies against pseudorabies virus and hog cholera virus and formed HCV-E1-specific antibodies.

It was furthermore determined whether vaccination of pigs with one of the three constructs PRV-M203, -M204 and -M205 leads to protection of these pigs against a virulent infection with hog cholera virus.

The test animals used were four groups (A-D) comprising 10 week old SPF pigs which had no antibodies against HCV and BVDV. Each test group comprised four pigs.

On day 0, the four pigs in group A were vaccinated intramuscularly with 10^{7.3} PFU of PRV-M203 (see Fig. 4), those in group B with 10^{7.3} PFU of PRV-M204, those in group C with 10^{7.3} PFU of PRV-M205, and those in group D with 10^{7.3} PFU of PRV-M206.

This vaccination was repeated on day 28. On day 42, all pigs in the four groups were infected intranasally with 100 LD₅₀ doses of the virulent HCV Brescia strain 456610 (Terpstra and Wensvoort, Vet. Microbiol. 16, 123-128 (1988)).

Serum blood samples were taken on days -3, 28, 42 and 63. The sera were examined in the serum neutralization test (SNT-PRV) (De Leeuw et al., Vet. Quarterly 4, 49-56 (1982)) to detect neutralizing antibodies against PRV. The sera were examined in the neutralizing peroxidase-linked assay (NPLA) (Terpstra at al., Vet. Microbiol. 9, 113-120 (1984)) to detect neutralizing antibodies against HCV. The sera were investigated further in the complex trapping blocking ELISA (CTB) (Wensvoort at al., Vet. Microbiol. 17, 129-140 (1988)) to detect HCV-E1-specific antibodies.

EDTA blood samples were taken on days 42, 45, 47, 49, 52 and 56, and 63. The number of erythrocytes, thrombocytes and leukocytes in these samples was counted. Furthermore, the leukocytes were isolated from these samples and the HCV titers of the leukocyte fractions were determined by titration of log-dilutions of the fractions in PK-15 cells. The cells were subsequently cultured for 4 days at 37°C and with 5% CO₂, after which the presence of virus was determined in an IPMA with monoclonal antibodies raised against HCV Brescia strain (Wensvoort et al., Vet. Microbiol. 12, 101-108 (1986)).

The rectal temperatures of all the pigs were measured daily and the pigs were examined daily for development of signs of disease.

**Table 1**

| | | | | | | | | | | | | |
|---|---|---|---|---|---|---|---|---|---|---|---|---|
| Antibody responses of groups A through D. Sera were taken on day -3, 28, 42, and 63, and tested in duplicate in the SNT-PRV, the NPLA, and the CTB. For the SNT-PRV en de NPLA , titers were expressed as the log of the reciprocal dilution that prevented the replication of 100-300 TCID50 virus. For the CTB, titers were expressed as the percentage inhibition of a standard negative signal (Wensvoort et al, Vet. Microbiol. 17, 129-140 (1988)). | | | | | | | | | | | | |

| Test day | SNT-PRV | | | | NPLA | | | | CTB | | | |
|---|---|---|---|---|---|---|---|---|---|---|---|---|
| | -3 | 28 | 42 | 63 | -3 | 28 | 42 | 63 | -3 | 28 | 42 | 63 |
| Group A (M203)* | | | | | # | | | | @ | | | |
| pig 1 | - | 1.4 | 4.2 | 5.7 | - | - | 1.4 | 3.7 | 8 | 5 | 62 | 90 |
| pig 2 | - | 1.4 | 4.2 | 3.9 | - | - | - | 2.7 | 8 | 0 | 30 | 89 |
| pig 3 | - | 1.4 | 3.9 | 3.2 | - | - | 2.4 | 4.3 | 3 | 0 | 87 | 92 |
| pig 4 | - | 1.8 | 4.5 | 4.8 | - | - | 1.5 | 4.2 | 13 | 0 | 48 | 91 |

| Group B (M204) | | | | | | | | | | | | |
|---|---|---|---|---|---|---|---|---|---|---|---|---|
| pig 5 | - | 1.4 | 5.9 | 4.2 | - | 1.3 | 3.1 | 4.5 | 6 | 36 | 94 | 90 |
| pig 6 | - | 2.0 | 4.8 | 4.8 | - | 1.5 | 3.3 | 5.0 | 2 | 35 | 92 | 90 |
| pig 7 | - | 1.5 | 4.2 | 3.0 | - | 1.7 | 3.3 | 5.0 | 5 | 42 | 94 | 91 |
| pig 8 | - | 1.5 | 4.5 | 2.7 | - | 1.9 | 3.4 | 5.0 | 0 | 43 | 91 | 93 |

| Group C (M205) | | | | | | | | | | | | |
|---|---|---|---|---|---|---|---|---|---|---|---|---|
| pig 9 | - | 1.8 | 4.2 | 4.2 | - | 1.1 | 3.1 | 3.1 | 2 | 11 | 86 | 86 |
| pig 10 | - | 1.5 | 4.8 | 3.3 | - | 1.6 | 3.4 | 5.0 | 5 | 23 | 89 | 92 |
| pig 11 | - | 1.8 | 4.1 | 3.9 | - | 1.3 | 3.7 | 3.4 | 1 | 16 | 94 | 89 |
| pig 12 | - | 1.4 | 3.0 | 2.7 | - | 1.3 | 3.4 | 5.0 | 4 | 21 | 94 | 91 |

| Group D (M206) | | | | | | | | | | | | |
|---|---|---|---|---|---|---|---|---|---|---|---|---|
| pig 13 | - | 2.1 | 4.2 | d | - | - | - | d | 7 | 0 | 0 | d |
| pig 14 | - | 2.3 | 3.9 | d | - | - | - | d | 6 | 0 | 0 | d |
| pig 15 | - | 2.1 | 4.2 | d | - | - | - | d | 4 | 15 | 0 | d |
| pig 16 | - | 2.3 | 4.4 | d | - | - | - | d | 0 | 8 | 0 | d |

| | | | | | | | | | | | | |
|---|---|---|---|---|---|---|---|---|---|---|---|---|
| * = <0.3, in the SNT-PRV these scores are considered negative; | | | | | | | | | | | | |
| # = <0.8, in the NPLA these scores are considered negative; | | | | | | | | | | | | |
| @ in the CTB a percentage inhibition of <30 is considered negative, while a percentage inhibition of >50 is considered positive. d = died | | | | | | | | | | | | |

**Table 2**

| | | | | | | |
|---|---|---|---|---|---|---|
| Hog cholera virus titers in leucocyte fractions of the pigs from group A, B, C, and D, after the pigs were inoculated on day 42 with 100 LD₅₀ of virulent hog cholera virus, strain Brescia 456610. | | | | | | |

| Day | 45 | 47 | 49 | 52 | 56 | 63 |
|---|---|---|---|---|---|---|
| Group A (M203) | * | | | | | |
| pig 1 | < | 0.72 | 0.88 | < | < | < |
| pig 2 | < | 0.72 | 4.05 | < | 0.72 | < |
| pig 3 | < | < | < | < | < | < |
| pig 4 | < | 0.72 | < | < | < | < |

| Group B (M204) | | | | | | |
|---|---|---|---|---|---|---|
| pig 5 | < | < | < | < | < | < |
| pig 6 | < | < | 0.72 | < | < | < |
| pig 7 | < | < | < | < | | |
| pig 8 | < | < | < | < | < | < |

| Group C (M205) | | | | | | |
|---|---|---|---|---|---|---|
| pig 9 | < | < | < | < | < | < |
| pig 10 | < | < | < | < | < | < |
| pig 11 | < | < | < | < | < | < |
| pig 12 | < | < | < | < | < | < |

| Group D (M206) | | | | | | |
|---|---|---|---|---|---|---|
| pig 13 | < | 2.55 | 5.55 | 4.38 | 5.05 | - |
| pig 14 | < | 2.05 | 5.05 | 5.38 | 5.82 | - |
| pig 15 | < | 3.28 | 6.05 | 4.55 | d | - |
| pig 16 | < | 2.38 | 5.72 | 4.72 | 5.55 | - |

| | | | | | | |
|---|---|---|---|---|---|---|
| * < = no virus detected; titers are expressed as log TCID50. d = died; pigs 13, 14, and 16 were sacrificed "in extremis" on day 56 - = not applicable | | | | | | |

**Table 3**

| | | | | | | | | | | | | | | | | |
|---|---|---|---|---|---|---|---|---|---|---|---|---|---|---|---|---|
| Means of clinical data of the pigs from group A, B, C, and D, after the pigs were included on day 42 with 100 LD₅₀ of virulent hog cholers virus, strain Brescia 456610. | | | | | | | | | | | | | | | | |

| day | 41 | 42 | 43 | 44 | 45 | 46 | 47 | 48 | 49 | 50 | 51 | 52 | 53 | 54 | 55 | 56 |
|---|---|---|---|---|---|---|---|---|---|---|---|---|---|---|---|---|
| Croup A (M203) | | | | | | | | | | | | | | | | |
| mean temp. * | 39.0 | 38.7 | 39.0 | 39.2 | 39.8 | 40.5 | 40.1 | 39.7 | 39.6 | 39.1 | 38.8 | 38.8 | 38.9 | 38.8 | 39.0 | 38.8 |
| (s. dev.) | 0.1 | 0.1 | 0.1 | 0.4 | 0.7 | 0 9 | 0.7 | 0.8 | 0.4 | 0.6 | 0.4 | 0.2 | 0.0 | 0.2 | 0.2 | 0.2 |
| | | | | | | | | | | | | | | | | |
| mean ery.# | | 6.2 | | | 6.2 | | 4.9 | | 5.0 | | | 5.5 | | | | 4.9 |
| (s.dev.) | | 0.2 | | | 0.3 | | 0.1 | | 0.2 | | | 0.3 | | | | 0.3 |
| mean thr.@ | | 318 | | | 263 | | 238 | | 264 | | | 298 | | | | 400 |
| (s.dev.) | | 45 | | | 69 | | 54 | | 84 | | | 111 | | | | 55 |
| mean leue.⁻ | | 10.1 | | | 5.6 | | 6.4 | | 6.3 | | | 9.5 | | | | 8.9 |
| s.dev.) | | 0.8 | | | 1.4 | | 1.6 | | 0.7 | | | 1.0 | | | | 0.1 |

| Group B (M204) | | | | | | | | | | | | | | | | |
|---|---|---|---|---|---|---|---|---|---|---|---|---|---|---|---|---|
| mean temp. | 39.0 | 38.8 | 38.9 | 38.8 | 39.3 | 39.2 | 39.1 | 39.1 | 39.6 | 40.0 | 39.2 | 39.1 | 39.0 | 38.8 | 39.2 | 39.1 |
| (s.dev.) | 0.1 | 0. 2 | 0.2 | 0.2 | 0.1 | 0.2 | 0.1 | 0.2 | 0.4 | 0.6 | 0.1 | 0.2 | 0.1 | 0.1 | 0.0 | 0.1 |
| | | | | | | | | | | | | | | | | |
| mean ery. | | 6.0 | | | 6.1 | | 5.2 | | 5.0 | | | 5.8 | | | | 5.1 |
| (s.dev.) | | 0.3 | | | 0.3 | | 0.1 | | 0.2 | | | 0.2 | | | | 0.2 |
| mean thr. | | 346 | | | 345 | | 288 | | 363 | | | 348 | | | | 353 |
| (s.dev.) | | 38 | | | 49 | | 25 | | 2 | | | 25 | | | | 19 |
| mean leue. | | 10.3 | | | 6.9 | | 7.9 | | 8.8 | | | 8.0 | | | | 8.4 |
| (s.dev.) | | 0.3 | | | 1.0 | | 1.0 | | 2.4 | | | 2.0 | | | | 0.9 |

| Croup C (M205) | | | | | | | | | | | | | | | | |
|---|---|---|---|---|---|---|---|---|---|---|---|---|---|---|---|---|
| mean temp. | 38.9 | 38.6 | 38.8 | 38.5 | 39.1 | 38.9 | 39.2 | 39.5 | 39.2 | 39.3 | 38.9 | 39.0 | 39.0 | 39.0 | 39.0 | 38.9 |
| (s.dev.) | 0.1 | 0.1 | 0.2 | 0.2 | 0.2 | 0.1 | 0.3 | 0.5 | 0.1 | 0.2 | 0.2 | 0.1 | 0.1 | 0.2 | 0.2 | 0.1 |
| | | | | | | | | | | | | | | | | |
| mean ery. | | 5.7 | | | 6.2 | | 5.3 | | 5.3 | | | 5.3 | | | | 4.7 |
| (s.dev.) | | 0.3 | | | 0.3 | | 0.3 | | 0.2 | | | 0.1 | | | | 0.4 |
| mean thr. | | 341 | | | 287 | | 307 | | 357 | | | 353 | | | | 397 |
| (s.dev.) | | 28 | | | 87 | | 38 | | 30 | | | 63 | | | | 58 |
| mean leve. | | 9.6 | | | 6.6 | | 9.0 | | 7.1 | | | 6.2 | | | | 8.1 |
| (s.dev.) | | 0.3 | | | 0.9 | | 1.5 | | 1.2 | | | 0.6 | | | | 0.5 |

| Group D (M206) | | | | | | | | | | | | | | | | |
|---|---|---|---|---|---|---|---|---|---|---|---|---|---|---|---|---|
| mean temp. | 39.0 | 38.9 | 39.0 | 39.4 | 40.8 | 40.9 | 41.2 | 41.3 | 41.5 | 41.7 | 41.4 | 41.1 | 40.8 | 40.5 | 40.0 | 38.6 |
| (s.dev.) | 0.1 | 0.2 | 0.2 | 0.4 | 0.3 | 0.2 | 0.3 | 0.4 | 0.3 | 0.4 | 0.2 | 0.3 | 0.2 | 0.7 | 0.6 | 0.7 |
| | | | | | | | | | | | | | | | | |
| mean ery. | | 5.4 | | | 6.0 | | 4.4 | | 5.1 | | | 3.4 | | | | 3.2 |
| (s.dev.) | | 0.1 | | | 0.2 | | 0.3 | | 0.2 | | | 0.6 | | | | 0.9 |
| mean thr. | | 332 | | | 265 | | 240 | | 129 | | | 50 | | | | 74 |
| (s.dev.) | | 12 | | | 77 | | 67 | | 68 | | | 20 | | | | 44 |
| mean leue. | | 8.1 | | | 4.9 | | 5.0 | | 3.3 | | | 4.2 | | | | 5.2 |
| (s.dev.) | | 1.1 | | | 1.0 | | 2.2 | | 0.6 | | | 1.6 | | | | 1.1 |

| | | | | | | | | | | | | | | | | |
|---|---|---|---|---|---|---|---|---|---|---|---|---|---|---|---|---|
| * mean temp.= The mean rectal temperature °C at approximately 10 a.m. | | | | | | | | | | | | | | | | |
| # mean ery. = The mean number of erythrocytes (times 10¹²) per liter | | | | | | | | | | | | | | | | |
| @ mean.thr. = The mean number of thrombocytes (times 10⁹) per liter | | | | | | | | | | | | | | | | |
| ⁻ mean.leue. = The mean number of leucocytes (times 10⁹) per liter | | | | | | | | | | | | | | | | |

### CONCLUSION

- Pigs vaccinated with one of the three constructs PRV-M203, M204 and M205 develop neutralizing antibodies against PRV and HCV and antibodies specific for HCV-E1. Pigs vaccinated with PRV-M206 develop neutralizing antibodies against PRV, but no neutralizing antibodies against HCV or antibodies specific for HCV-E1 (Table 1). Compound with PRV-M206, the sole additional genetic information contained in the three PRV-HCV constructs is the genome sequence which codes for HCV-E1 or sections of HCV-E1: this means that the pig sera directed against HCV-E1 can neutralize HCV.
- Pigs vaccinated with PRV-M203 are partially protected against virulent HCV infection. Pigs vaccinated with the PRV-HCV constructs M204 or M205 are completely protected against virulent HCV infection.

Pigs vaccinated with PRV-M206 are not protected against virulent HCV infection (Tables 2 and 3). Pigs can therefore be protected against virulent hog cholera virus infection by vaccination with a vaccine containing the genetic information for HCV-E1 or parts of HCV-E1 or by vaccination with a vaccine in which the genetic product of HCV-E1 or parts of HCV-E1 is used as the only polypeptide against which an antibody response to HCV is generated, or by vaccination with polypeptides which are derived in some way from the genetic information which codes for HCV-E1 or parts of HCV-E1.

## Claims

1. Nucleotide sequence encoding a part of the amino acid sequence of the hog cholera virus (HCV) Brescia strain, said part comprising one or more of the amino acid sequences 1-1148, 1-267, 268-570, 571-690, 691-1148, 691-1030, 1012-1031, 1012-1063, 1031-1148 and 1031-1063 of the amino acid sequence shown in Fig. 1.

2. Nucleotide sequence comprising a part of the genome of a pestivirus, said part encoding a polypeptide consisting of an amino acid sequence corresponding to the amino acid sequences 268-570, 691-1148, 691-1030 or 1031-1063 as shown in Figure 1.

3. Recombinant polynucleotide containing a nucleotide sequence according to Claim 1 or 2.

4. Recombinant polynucleotide according to Claim 3, wherein the sequence is contained in a pseudorabies virus vector.

5. Expression system containing a recombinant polynucleotide according to any one of Claims 3-4 and capable of expressing the nucleotide sequence.

6. Recombinant polypeptide comprising a part of the amino acid sequence of the hog cholera virus (HCV) Brescia strain, said part having one of the amino acid sequences 1-1148, 1-267, 268-570, 571-690, 691-1148, 691-1030, 1012-1031, 1012-1063, 1031-1148 and 1031-1063 of the amino acid sequence shown in Fig. 1, the polypeptide optionally being glycosylated.

7. Recombinant polypeptide comprising a part of the amino acid sequence of the hog cholera virus (HCV), said part consisting of an amino acid sequence corresponding to the amino acid sequences 268-570, 691-1148, 691-1030 or 1031-1063 as shown in Figure 1.

8. A process for preparing a recombinant polypeptide comprising a part of the amino acid sequence of the HCV Brescia strain, comprising the step of expressing an amino acid sequence comprising one of the amino acid sequences 1-267, 268-570, 571-690, 691-1148, 691-1030, 1012-1031, 1012-1063, 1031-1148 and 1031-1063 of Fig. 1, using DNA encoding said amino acid sequence to be expressed.

9. A process for preparing a recombinant polypeptide comprising a part of the amino acid sequence of the hog cholera virus (HCV), whereby said part consists of an amino acid sequence corresponding to the amino acid sequences 268-570, 691-1148, 691-1030 or 1031-1063 as shown in Figure 1, the process comprising the step of expressing the polypeptide using DNA encoding said polypeptide to be expressed.

10. Vaccine for protection against a pestivirus infection, containing a nucleotide sequence according to Claim 1 or 2.

11. Vaccine for protection against a pestivirus infection, containing a polypeptide produced according to Claim 6 or 7, or a polypeptide prepared according to claim 8 or 9.

12. Diagnostic kit, which kit contains at least a polypeptide produced according to Claim 6 or 7, or a polypeptide prepared according to claim 8 or 9.

13. Diagnostic kit according to Claim 12 for diagnosing hog cholera, containing a polypeptide comprising an amino acid sequence corresponding to the amino acid sequence 268-570 of Fig. 1.

## Patentansprüche

1. Nucleotidsequenz, die einen Teil der Aminosäuresequenz von dem Schweine-Cholera-Virus (HCV) Stamm Brescia codiert, wobei dieser Teil eine oder mehrere Aminosäuresequenzen 1-1148, 1-267, 268-570, 571-690, 691-1148, 691-1030, 1012-1031, 1012-1063, 1031-1148 und 1031-1063 der in Figur 1 gezeigten Aminosäuresequenz umfaßt.

2. Nucleotidsequenz, umfassend einen Teil des Genoms eines Pestivirus, wobei der Teil, der ein Polypeptid codiert, aus einer Aminosäuresequenz besteht, die den in Figur 1 gezeigten Aminosäuresequenzen 268-570, 691-1148, 691-1030 oder 1031-1063, entspricht.

3. Rekombinantes Polynucleotid, enthaltend eine Nucleotidsequenz gemäß Anspruch 1 oder 2.

4. Rekombinantes Polynucleotid nach Anspruch 3, wobei die Sequenz einen Pseudo-Tollwutvirusvektor enthält.

5. Expressionssystem, das ein rekombinantes Polynucleotid nach einem der Ansprüche 3 bis 4 enthält, und das die Nucleotidsequenz exprimieren kann.

6. Rekombinantes Polypeptid, umfassend einen Teil der Aminosäuresequenz des Schweine-Cholera-Virus (HCV) vom Stamm Brescia, wobei dieser Teil eine der Aminosäuresequenzen 1-1148, 1-267, 268-570, 571-690, 691-1148, 691-1030, 1012-1031, 1012-1063, 1031-1148 und 1031-1063 der in Figur 1 gezeigten Aminosäuresequenz aufweist, wobei das Polypeptid wahlweise glycosyliert ist.

7. Rekombinantes Polypeptid, umfassend einen Teil der Aminosäuresequenz des Schweine-Cholera-Virus (HCV), wobei der Teil aus den in Figur 1 gezeigten Aminosäuresequenzen 268-570, 691-1148, 691-1030 oder 1031-1063, besteht.

8. Verfahren zur Herstellung eines rekombinanten Polypeptids, das einen Teil der Aminosäuresequenz des HCV Stammes Brescia umfasst, umfassend den Schritt des Exprimierens einer Aminosäuresequenz, die eine der Aminosäuresequenzen 1-267, 268-570, 571-690, 691-1148, 691-1030, 1012-1031, 1012-1063, 1031-1148 und 1031-1063 der Figur 1 umfaßt, unter Verwendung einer DNA, die die zu exprimierende Aminosäuresequenz codiert.

9. Verfahren zur Herstellung eines rekombinanten Polypeptids, das einen Teil der Aminosäuresequenz von dem Schweine-Cholera-Virus (HCV) umfaßt, wobei der Teil aus einer Aminosäuresequenz besteht, die den in Figur 1 gezeigten Aminosäuresequenzen 268-570, 691-1148, 691-1030 oder 1031-1063 entspricht, und das Verfahren den Schritt des Exprimierens des Polypeptids unter Verwendung einer DNA, die das zu exprimierende Polypeptid codiert, umfasst.

10. Vakzine zum Schutz gegen eine Pestivirus-Infektion, die eine Nucleotidsequenz nach Anspruch 1 oder 2 enthält.

11. Vakzine zum Schutz gegen eine Pestivirus-Infektion, die ein nach Anspruch 6 oder 7 hergestelltes Polypeptid oder ein nach Anspruch 8 oder 9 hergestelltes Polypeptid enthält.

12. Diagnostisches Kit, wobei das Kit zumindest ein Polypeptid, das nach Anspruch 6 oder 7 hergestellt wird, oder ein Polypeptid, das nach Anspruch 8 oder 9 hergestellt wird, enthält.

13. Diagnostisches Kit nach Anspruch 12 zur Diagnose von Schweine-Cholera, enthaltend ein Polypeptid, das eine Aminosäuresequenz umfaßt, die der Aminosäuresequenz 268-570 der Figur 1 entspricht.

## Revendications

1. Séquence nucléotidique codant pour une partie de la séquence d'acides aminés de la souche Brescia du virus de la peste porcine (HCV), ladite partie comprenant une ou plusieurs des séquences d'acides aminés 1-1148, 1-267, 268-570, 571-690, 691-1148, 691-1030, 1012-1031, 1012-1063, 1031-1148 et 1031-1063 de la séquence d'acides aminés présentée à la figure 1.

2. Séquence nucléotidique comprenant une partie du génome d'un pestivirus, ladite partie codant pour un polypeptide comprenant une séquence d'acides aminés correspondant aux séquences d'acides aminés 268-570, 691-1148, 691-1030 ou 1031-1063 présentées à la figure 1.

3. Polynucléotide recombinant contenant une séquence nucléotidique selon la revendication 1 ou 2.

4. Polynucléotide recombinant selon la revendication 3, dans lequel la séquence est contenue dans un vecteur du virus de pseudorage.

5. Système d'expression contenant un polynucléotide recombinant selon l'une quelconque des revendications 3 à 4 et apte à exprimer la séquence nucléotidique.

6. Polypeptide recombinant comprenant une partie de la séquence d'acides aminés de la souche Brescia du virus de la peste porcine (HCV), ladite partie ayant l'une des séquences d'acides aminés 1-1148, 1-267, 268-570, 571-690, 691-1148, 691-1030, 1012-1031, 1012-1063, 1031-1148 et 1031-1063 de la séquence d'acides aminés présentée à la figure 1, le polypeptide étant éventuellement glycosylé.

7. Polypeptide recombinant comprenant une partie de la séquence d'acides aminés du virus de la peste porcine (HCV), ladite partie consistant en une séquence d'acides aminés 268-570, 691-1148, 691-1030 ou 1031-1063 présentée à la figure 1.

8. Procédé pour préparer un polypeptide recombinant comprenant une partie de la séquence d'acides aminés de la souche Brescia du HCV, comprenant l'étape consistant à exprimer une séquence d'acides aminés comprenant l'une des séquences d'acides aminés 1-267, 268-570, 571-690, 691-1148, 691-1030, 1012-1031, 1012-1063, 1031-1148 et 1031-1063 de la figure 1, en utilisant un ADN codant pour ladite séquence d'acides aminés à exprimer.

9. Procédé pour préparer un polypeptide recombinant comprenant une partie de la séquence d'acides aminés du virus de la peste porcine (HCV), par lequel ladite partie consiste en une séquence d'acides aminés correspondant aux séquences d'acides aminés 268-570, 691-1148, 691-1030 ou 1031-1063 présentées à la figure 1, le procédé comprenant l'étape consistant à exprimer le polypeptide en utilisant un ADN codant pour ledit polypeptide à exprimer.

10. Vaccin pour la protection contre une infection par un pestivirus, contenant une séquence nucléotidique selon la revendication 1 ou 2.

11. Vaccin pour la protection contre une infection par un pestivirus, contenant un polypeptide selon la revendication 6 ou 7, ou un polypeptide préparé selon la revendication 8 ou 9.

12. Nécessaire de diagnostic, lequel contient au moins un polypeptide selon la revendications 6 ou 7, ou un polypeptide préparé selon la revendication 8 ou 9.

13. Nécessaire de diagnostic selon la revendication 12 pour diagnostiquer la peste porcine, contenant un polypeptide comprenant une séquence d'acides aminés correspondant à la séquence d'acides aminés 268-570 de la figure 1.
